# FASCICULE DE BREVET EUROPEEN

(11) **EP 0 671 158 B1**
(45) Date de publication et mention de la délivrance du brevet: **21.04.1999**
(21) Numéro de dépôt: 95400186.3
(22) Date de dépôt: 27.01.1995
(51) Int. Cl.: A61K 7/09

(54) **Procédé pour la déformation permanente des matières kératiniques**
Verfahren zur Dauerhaften Verformung von Keratinsubstanzen
Permanent wave process for keratinic fibres

(30) Priorité: 07.03.1994 FR 9402585
(43) Date de publication de la demande: 13.09.1995
(73) Titulaire: L'OREAL, 75008 Paris (FR)
(72) Inventeur: Samain, Henri, F-91570 Bievres (FR)
(74) Mandataire: Tezier Herman, Béatrice

(56) Documents cités:
- EP-A- 0 551 135
- FR-A- 2 486 395

## Description

La présente invention concerne un nouveau procédé de traitement des matières kératiniques, en particulier des cheveux, en vue d'obtenir une déformation permanente de ces dernières, en particulier sous la forme de cheveux permanentés, ledit procédé étant notamment utilisable dans le domaine des salons de coiffure, de beauté, de cosmétique et analogues, professionnels.

On sait que la technique la plus usuelle pour obtenir une déformation permanente des cheveux consiste, dans un premier temps, à réaliser l'ouverture des liaisons disulfures -S-S- de la kératine (cystine) à l'aide d'une composition contenant un agent réducteur (étape de réduction) puis, après avoir de préférence rincé la chevelure ainsi traitée, à reconstituer dans un second temps lesdites liaisons disulfures en appliquant, sur les cheveux préalablement mis sous tension (bigoudis et autres), une composition oxydante (étape d'oxydation, dite aussi de fixation) de façon à donner finalement aux cheveux la forme recherchée. Cette technique permet ainsi de réaliser indifféremment soit l'ondulation des cheveux, soit leur défrisage ou leur décrêpage. La nouvelle forme imposée aux cheveux par un traitement chimique tel que ci-dessus est éminemment durable dans le temps et résiste notamment à l'action des lavages à l'eau ou par shampooings, et ceci par opposition aux simples techniques classiques de déformation temporaire, telles que de mise en pli.

La demande de brevet EP 551 135 concerne l'étape de réduction des fibres kératiniques au moyen d'une solution réductrice aqueuse comprenant un dérivé de cystéamine et l'utilisation de dérivés thioglycoliques est de préférence écartée. Une composition oxydante classique est ensuite utilisée pour maintenir durablement la permanente.

La demande de brevet FR 2 486 395 décrit une composition pour l'ondulation des cheveux en une seule étape, et qui comprend, comme ingrédient en solution principale, un sulfure, un hydrosulfure ou un mercaptan, un accélérateur d'oxydation à l'air, des alcalis libres pour régler le pH et un support aqueux.

Les compositions réductrices utilisables pour la mise en oeuvre de la première étape d'une opération de permanente contiennent généralement, à titre d'agents réducteurs, des sulfites, des bisulfites ou des thiols. Parmi ces derniers, on peut citer la cystéïne et ses divers dérivés, la cystéamine et ses dérivés, l'acide thiolactique, l'acide thioglycolique ainsi que ses esters, notamment le monothioglycolate de glycérol, et le thioglycérol. A cet égard, et bien que possédant une odeur désagréable, l'acide thioglycolique est particulièrement efficace, et constitue ainsi le composé de référence en permanente pour réduire les liaisons disulfures de la kératine la cystéïne, quant à elle, produit une odeur beaucoup plus faible que celle de l'acide thioglycolique ou du monothioglycolate de glycérol, mais le degré de frisure obtenu est malheureusement inférieur et loin d'être totalement satisfaisant.

Concernant les compositions oxydantes nécessaires à la mise en oeuvre de l'étape de fixation, on fait le plus souvent appel, dans la pratique, à des compositions à base d'eau oxygénée. Or, il s'avère que l'emploi d'eau oxygénée présente notamment pour inconvénient de conduire à une dégradation plus ou moins marquée de la couleur originelle de la chevelure.

En outre, et en particulier dans le cas où l'agent réducteur utilisé est l'acide thioglycolique, on observe que la succession des cycles réduction - oxydation (i.e. d'opérations de permanente) sur le cheveu conduit, de manière néfaste, à une dégradation progressive non seulement de la couleur de ce dernier (décoloration) mais également de sa tenue mécanique (diminution de l'énergie de rupture) en particulier dûe à une augmentation significative du taux d'acide kératocystéïque dans le cheveu traité.

La présente invention a notamment pour but de résoudre les problèmes ci-dessus.

Plus précisément encore, la présente invention a pour but de proposer un nouveau procédé de traitement convenant à la déformation permanente des matières kératiniques et qui permette de s'affranchir de la mise en oeuvre des étapes classiques de fixation par des agents oxydants puissants.

Elle a également pour but de proposer un procédé tel que ci-dessus qui permette en outre d'obtenir des frisures de haute qualité.

Elle a également pour but de proposer un procédé tel que ci-dessus qui permette de limiter, voire supprimer, la dégradation mécanique du cheveu, après répétition du traitement.

Elle a aussi pour but de proposer un procédé tel que ci-dessus, limitant, voire supprimant, la décoloration du cheveu.

Elle a enfin pour but de proposer un procédé tel que ci-dessus qui soit, globalement, peu odorant d'une part, et peu irritant pour la peau et/ou le cuir chevelu d'autre part.

Or, il a été trouvé par la demanderesse que ces buts, et d'autres, pouvaient être atteints avec succès en procédant à une sélection convenable de la composition dite réductrice de départ associée à un mode opératoire particulier de mise en oeuvre de cette composition. Cette découverte est à la base de la présente invention.

Ainsi, il est maintenant proposé selon la présente invention un nouveau procédé de traitement convenant à la déformation et/ou la mise en forme, et ceci de manière permanente, des matières kératiniques, et en particulier des cheveux ledit procédé étant caractérisé par le fait qu'il comprend les étapes suivantes :
(i) on applique sur la matière kératinique à traiter une composition contenant de l'acide thioglycolique et/ou de l'acide thiolactique et/ou de l'acide mercaptopropionique et/ou un de leurs sels et/ou un de leurs esters, les moyens (tels que par exemple rouleaux, bigoudis et analogues) nécessaires à la mise sous tension mécanique de la matière kératinique étant mis en oeuvre avant pendant ou après ladite application,
(ii) éventuellement, on soumet ensuite la matière kératinique ainsi traitée à un traitement thermique,
(iii) puis on rince la matière kératinique ainsi traitée,
(iv) on laisse ensuite reposer la matière kératinique ainsi rincée,
(v) et enfin on sépare la matière kératinique ainsi reposée des moyens (rouleaux et autres) de mise sous tension utilisés à l'étape (i).

Le procédé selon l'invention convient particulièrement bien à l'obtention d'une chevelure permanentée.

Appliqué sur une chevelure saine, et même répété plusieurs fois, le procédé selon l'invention présente pour avantages principaux, entre autres, de conduire et ceci sans dégagement d'odeurs désagréables d'une part et d'une façon non irritante pour la peau et/ou le cuir chevelu d'autre part, à des cheveux non décolorés ou substantiellement non décolorés, résistants mécaniquement, et présentant de belles frisures.

Mais d'autres caractéristiques, aspects et avantages de l'invention apparaîtront encore plus clairement à la lecture de la description détaillée qui va suivre, ainsi que des divers exemples concrets, mais nullement limitatifs, destinés à l'illustrer.

Bien que l'exposé qui suit s'articule essentiellement autour du cas particulier du traitement du cheveu, on notera ici que le procédé selon l'invention est applicable à toute matière kératinique en général, notamment cils, moustaches, poils, laine et autres.

Les agents réducteurs utilisés dans le cadre du procédé selon l'invention sont l'acide thioglycolique, de formule (1) :

HS-CH₂-COOH (1)

l'acide thiolactique, de formule (2) : 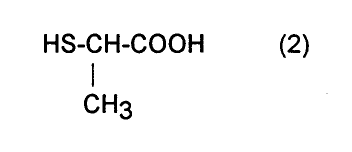 l'acide 3-mercaptopropionique, de formule (3) :

HS-CH₂-CH₂-COOH (3)

ou un de leurs sels ou un de leurs esters.

Parmi les sels cosmétiquement acceptables des produits (1), (2) et (3) ci-dessus, on peut plus particulièrement mentionner les sels d'ammonium, les sels d'amine primaire, secondaire ou tertiaire, les sels de métaux alcalino-terreux. Comme sels d'amine primaire, secondaire ou tertiaire, on peut citer respectivement la monoéthanolamine, la di-isopropanolamine et la triéthanolamine.

Parmi les esters des composés (1), (2) et (3) ci-dessus, on peut citer le monothioglycolate de glycérol, le monothioglycolate d'éthylène glycol, le mélange azéotrope de thioglycolate d'hydroxy-2-propyle et de thioglycolate d'hydroxy-2-méthyl-1 éthyle décrit dans la demande de brevet FR-A-2 679 448, le monothiolactate de glycérol, le monothiolactate d'éthylène glycol, le 3-mercaptopropionate de glycérol, le 3-mercaptopropionate d'éthylène glycol.

Ces agents réducteurs sont généralement mis en oeuvre dans des compositions cosmétiquement acceptables, lesquelles sont par ailleurs déjà bien connues en soi dans l'état de l'art existant des formulations frisantes destinées à réaliser la première étape (réduction) d'une opération de permanente. Ainsi, à titre d'additifs usuels et classiques, utilisables seuls ou en mélanges, on peut plus particulièrement mentionner les agents tensioactifs de type non-ionique, anionique, cationique ou amphotère et parmi ceux-ci, on peut citer les alkylsulfates, les alkylbenzènesulfates, les alkyléthersulfates, les alkylsulfonates, les sels d'ammonium quaternaire, les alkylbétaïnes, les alkylphénols oxyéthylénés, les alcanolamides d'acides gras, les esters d'acides gras oxyéthylénés ainsi que d'autres tensioactifs non-ioniques du type hydroxypropyléthers.

Lorsque la composition réductrice contient au moins un agent tensioactif, celui-ci est généralement présent à une concentration maximale de 30 % en poids, et de préférence comprise entre 0,5 et 10% en poids, par rapport au poids total de la composition réductrice.

Dans le but d'améliorer les propriétés cosmétiques des cheveux ou encore d'en atténuer ou d'éviter leur dégradation, la composition réductrice peut également contenir un agent traitant de nature cationique, anionique, non-ionique ou amphotère.

Parmi les agents traitants particulièrement préférés, on peut notamment citer ceux décrits dans les demandes de brevets français n° 2 598 613 et 2 470 596. On peut également utiliser comme agents traitants des silicones volatiles ou non, linéaires ou cycliques et leurs mélanges, les polydiméthylsiloxanes, les polyorganosiloxanes quaternisés tels que ceux décrits dans la demande de brevet français n° 2 535 730, les polyorganosiloxanes à groupements aminoalkyles modifiés par des groupements alcoxycarbonyalkyles tels que ceux décrits dans le brevet US n° 4 749 732, des polyorganosiloxanes tels que le copolymère polydiméthylsiloxane-polyoxyalkyle du type Diméthicone Copolyol, un polydiméthylsiloxane à groupements terminaux stéaroxy- (stéaroxydiméthicone), un copolymère polydiméthylsiloxane-dialkylammonium acétate ou un copolymère polydiméthyl-siloxane polyalkylbétaïne décrits dans la demande de brevet britannique n° 2 197 352, des polysiloxanes organo modifés par des groupements mercapto ou mercaptoalkyles tels que ceux décrits dans le brevet français n° 1 530 369 et dans la demande de brevet européen n° 295 780, ainsi que des silanes tels que le stéaroxytriméthylsilane.

La composition réductrice peut également contenir d'autres ingrédients traitants tels que des polymères cationiques tels que ceux utilisés dans les compositions des brevets français n° 79.32078 (FR-A- 2 472 382) et 80.26421 (FR-A- 2 495 931), ou encore des polymères cationiques du type ionène tels que ceux utilisés dans les compositions du brevet luxembourgeois n° 83703, des aminoacides basiques (tels que la lysine, l'arginine) ou acides (tels que l'acide glutamique, l'acide aspartique), des peptides et leurs dérivés, des hydrolysats de protéines, des cires, des agents de gonflement et de pénétration ou permettant de renforcer l'efficacité du réducteur tels que le mélange SiO2/PDMS (polydiméthylsiloxane), le diméthylisosorbitol, I'urée et ses dérivés, la pyrrolidone, les N-alkyl-pyrrolidones, la thiomorpholinone, les alkyléthers d'alkylèneglycol ou de dialkylèneglycol tels que par exemple le monométhyléther de propylène glycol, le monométhyléther de dipropylène glycol, le monoéthyléther de l'éthylèneglycol et le monoéthyléther du diéthylèneglycol, des alcanediols en C₃-C₆ tels que par exemple le propanediol-1,2 et le butanediol-1,2, l'imidazolidinone-2 ainsi que d'autres composés tels que des alcools gras, des dérivés de la lanoline, des ingrédients actifs tels que l'acide panthothénique, des agents antichute, des agents antipelliculaires, des épaississants, des agents de suspension, des agents séquestrants, des agents opacifiants, des colorants, des filtres solaires ainsi que des parfums et des conservateurs.

Lorsque l'on utilise l'acide thioglycolique ou l'acide thiolactique ou l'acide 3-mercaptopropionique, ou l'un de leurs sels, à titre d'agent réducteur, le pH de l'ensemble de la composition réductrice est de préférence compris entre 6,5 et 11,5 et encore plus préférentiellement entre 7 et 10.

Lorsque l'on utilise les esters de l'acide thioglycolique ou de l'acide thiolactique ou de l'acide 3-mercaptopropionique, à titre d'agent réducteur, le pH de l'ensemble de la composition réductrice est de préférence compris entre 5 et 11 et encore plus préférentiellement entre 6 et 10.

Ces pH peuvent être obtenus et/ou ajustés classiquement par ajout soit d'agents basifiants, tels que par exemple l'ammoniaque, la monoéthanolamine, la diéthanolamine, la triéthanolamine, l'isopropanolamine, la di-isopropanolamine, la propanediamine-1,3, un carbonate ou bicarbonate alcalin ou d'ammonium, un carbonate organique tel que le carbonate de guanidine, ou bien encore un hydroxyde alcalin, tous ces composés pouvant bien entendu être pris seuls ou en mélange, soit d'agents acidifiants tels que par exemple l'acide chlorhydrique, l'acide acétique, l'acide lactique ou l'acide borique.

Dans les compositions réductrices de permanente utilisables dans le cadre de l'invention, les agents réducteurs mentionnés ci-avant sont généralement présents à une concentration qui peut être comprise entre 1 et 30 % en poids, et de préférence entre 3 et 20 % en poids par rapport au poids total de la composition réductrice.

La composition réductrice peut se présenter sous la forme d'une lotion, épaissie ou non, d'une crème, d'un gel, ou de toute autre forme appropriée.

La composition réductrice peut être également du type exothermique, c'est-à-dire provoquant un certain échauffement lors de l'application sur les cheveux, ce qui apporte un agrément à la personne qui subit la permanente ou le défrisage.

La composition réductrice peut également contenir un solvant tel que par exemple de l'éthanol, du propanol, ou de l'isopropanol ou encore du glycérol à une concentration maximale de 20 % par rapport au poids total de la composition.

Le véhicule des compositions est de préférence l'eau ou une solution hydroalcoolique d'un alcool inférieur tel que l'éthanol, l'isopropanol ou le butanol.

Lorsque les compositions sont destinées à une opération de défrisage ou de décrêpage des cheveux, la composition réductrice est de préférence sous forme d'une crème épaissie de façon à maintenir les cheveux aussi raides que possible. On réalise ces crèmes, sous forme d'émulsions "lourdes", par exemple à base de stéarate de glycéryle, de stéarate de glycol, de cires auto-émulsionnables, d'alcools gras, etc.

On peut également utiliser des liquides ou des gels contenant des agents épaississants tels que des polymères ou des copolymères carboxyvinyliques qui "collent" les cheveux et les maintiennent dans la position lissée pendant le temps de pose.

Enfin, les compositions peuvent être également sous forme dite "auto-neutralisante" ou encore "auto-régulée" et dans ce cas, les agents réducteurs de formule générale (1) et (2) sont associés à au moins un disulfure connu pour son utilisation dans une composition réductrice pour permanente auto-neutralisante.

Parmi de tels disulfures connus, on peut notamment mentionner l'acide dithioglycolique, le dithioglycérol, la cystamine, la N, N'-diacétyl-cystamine, la cystine, la pantéthine, et les disulfures des N-(mercapto-alkyl)ω-hydroxyalkylamides décrits dans la demande de brevet EP-A-354 835, les disulfures des N-mono ou N,N-dialkylmercapto-4 butyramides décrits dans la demande de brevet EP-A-368 763, les disulfures des aminomercapto-alkylamides décrits dans la demande de brevet EP-A-432 000, les disulfures des dérivés des acides N-(mercaptoalkyl)-succinamiques ou des N-(mercaptoalkyl)-succinimides décrits dans la demande de brevet EP-A-465 342, et les disulfures des alkylaminomercaptoalkylamides décrits dans la demande de brevet EP-A-514 282. Ces disulfures sont généralement présents dans un rapport molaire de 0,5 à 2,5, et de préférence de 1 à 2, par rapport à l'agent réducteur (voir brevet US 3 768 490).

Conformément à la première étape du procédé selon la présente invention (étape (i)), les compositions contenant le ou les agents réducteurs de formules (1), (2) ou (3) données ci-avant, ou un de leurs sels ou un de leurs esters, sont alors appliquées sur les cheveux à traiter, lesquels auront été de préférence préalablement mouillés.
Cette application peut être réalisée avant, pendant ou après l'habituelle étape de mise sous tension des cheveux sous une forme correspondant à la forme finale désirée pour ces derniers (boucles par exemple), cette étape pouvant elle-même être mise en oeuvre par tout moyen, mécanique notamment, approprié et connu en soi pour maintenir sous tension des cheveux, tels que par exemple rouleaux, bigoudis et analogues.

Selon une étape facultative du procédé de l'invention, (étape (ii)), on peut, après application de la composition réductrice, soumettre la chevelure à un traitement thermique, c'est-à-dire un chauffage. Dans ce cas, la température de chauffage est généralement comprise entre 30 et 60°C. Bien que non obligatoire, ce chauffage est néanmoins préféré, car il permet d'ajuster à volonté le degré final de frisure du cheveu. Bien entendu, un travail à température ambiante est possible, et n'est donc pas exclu du cadre de l'invention. Dans la pratique, cette opération peut être conduite au moyen d'un casque de coiffure, d'un sèche cheveux, d'un dispensateur de rayons infra-rouges, ou de tout autre appareil chauffant classique.

Avant de procéder à l'étape suivante de rinçage, il convient, de manière classique, de laisser reposer pendant quelques minutes, généralement entre 2 et 30 mn, de préférence entre 5 et 20 mn, la chevelure sur laquelle a été appliquée la composition réductrice, et ceci de façon à bien laisser le temps au réducteur d'agir correctement sur les cheveux; pendant cette phase d'attente, qui intègre l'étape facultative de chauffage mentionnée ci-avant, on prend soin que les cheveux ne sèchent pas complètement et restent ainsi humides jusqu'au moment de la mise en oeuvre de l'étape suivante (utilisation possible de bonnets, de gels de protection par exemple).

Dans une troisième étape indispensable du procédé selon l'invention (étape (iii)), les cheveux imprégnés de composition réductrice sont ensuite rincés soigneusement, généralement à l'eau.

Selon une quatrième étape absolument essentielle du procédé de traitement selon l'invention (étape (iv)), les cheveux ainsi rincés sont ensuite laissés dans une phase de repos ou d'attente. Si l'on supprime cette étape, il n'est pas possible d'obtenir de frisure correcte. Selon l'invention, cette phase de repos (ou d'attente) des cheveux rincés peut durer entre 3 et 60 mn, et elle est de préférence comprise entre 5 et 30 mn. Elle est généralement conduite en laissant reposer à l'air libre (température ambiante) les cheveux mouillés issus du rinçage. Une phase d'attente conduite à température plus élevée n'est pas exclue, et convient également. On notera que cette étape peut être conduite jusqu'à l'obtention de cheveux parfaitement secs, le procédé selon l'invention s'apparentant alors dans ce cas (sauf au niveau du résultat) aux procédés dits de "mise en plis".

Enfin, dans la dernière étape du procédé selon l'invention (étape (v)), on enlève de la chevelure les moyens mécaniques (rouleaux, bigoudis et analogues) qui maintenaient sous tension et dans la forme désirée les cheveux tout au long du traitement, ce par quoi l'on peut obtenir finalement une chevelure présentant par exemple de belles boucles permanentes (en particulier résistantes à l'eau), et ceci sans avoir eu à mettre en oeuvre d'étape chimique de fixation (oxydation).

Hormis le cas particulier du procédé type "mise en plis" évoqué ci-dessus, on notera que, avant ou après la mise en oeuvre de l'étape (v) ci-dessus (retrait des moyens mécaniques de mise sous tension du cheveu), il est possible de mettre en oeuvre une nouvelle étape de rinçage à l'eau des cheveux. Lorsque le retrait des rouleaux est réalisé sur des cheveux déja parfaitement secs, la mise en oeuvre d'une nouvelle étape de rinçage s'avère importante si l'on veut espérer obtenir une belle frisure.

Dans le procédé selon l'invention, lorsque la composition réductrice contient de l'acide thioglycolique, de l'acide thiolactique ou de l'acide mercaptopropionique ou leurs sels à une concentration supérieure à 8 % en poids par rapport au poids total de la composition, le pH de la composition réductrice est de préférence inférieure à 9 et la phase d'attente est inférieur à 7 minutes.

Des exemples concrets illustrant l'invention vont maintenant être donnés. Aux fins d'une comparaison significative, les mêmes cheveux de départ (avant traitement) ont été utilisés pour tous les exemples.

### Exemple 1 (invention)

On utilise une composition réductrice présentant les caractéristiques suivantes :

| | |
|---|---|
| - acide thioglycolique | 3, 6 g |
| - monoéthanolamine qs | pH 9,5 |
| - eau déminéralisée qsp | 100 g |

Le mode opératoire est le suivant : on applique sur des cheveux enroulés et humides (diamètre des rouleaux : 9 mm) la composition réductrice ci-dessus ; puis on pose un bonnet en plastique sur la chevelure (ce qui permet d'éviter le séchage des cheveux pendant l'étape suivante de chauffage) qui est alors installée sous un dispensateur d'infrarouges (40 °C) pendant 15 minutes ; puis on enlève le bonnet et on rince abondamment et soigneusement à l'eau ; on laisse ensuite les cheveux reposer sur les rouleaux pendant 15 minutes ; puis on retire les rouleaux (déroulage) ; enfin, on rince une nouvelle fois à l'eau.

On obtient ainsi finalement une mèche présentant une belle frisure.

### Exemple 2 (invention)

On utililise une composition réductrice présentant les caractéristiques suivantes :

| | |
|---|---|
| - acide thioglycolique | 9,1 g |
| - monoéthanolamine | 4 g |
| - bicarbonate d'ammonium | 2 g |
| - ammoniaque qs | pH 8,5 |
| - eau déminéralisée qsp | 100 g |

Le mode opératoire est celui de l'exemple 1.

On obtient une mèche présentant une belle frisure.

### Exemple 3 (invention)

On utilise une composition réductrice présentant les caractéristiques suivantes :

| | |
|---|---|
| - acide thioglycolique | 9, 1 g |
| - monoéthanolamine qs | pH 9,5 |
| - eau déminéralisée qsp | 100 g |

Le mode opératoire est celui de l'exemple 1, à l'exception du fait que les temps de pause des deux étapes sont limitées à 5 minutes par étape.

On obtient une mèche présentant une belle frisure.

### Exemple 4 (invention)

On utilise une composition réductrice présentant les caractéristiques suivantes :

| | |
|---|---|
| - acide thioglycolique | 9,1 g |
| - monoéthanolamine qs | pH 7 |
| - eau déminéralisée qsp | 100 g |

Le mode opératoire est celui de l'exemple 1.

On obtient une mèche présentant une belle frisure.

### Exemple 5 (invention)

On utilise une composition réductrice présentant les caractéristiques suivantes :

| | |
|---|---|
| - monothioglycolate de glycérol en solution à 68 % en poids dans le glycérol | 17 g MA |
| - monoéthanolamine qs | pH 9 |
| - eau déminéralisée qsp | 100 g |

Le mode opératoire est celui de l'exemple 1.

On obtient une mèche présentant une belle frisure.

### Exemple 6 (invention)

On utilise une composition réductrice présentant les caractéristiques suivantes :

| | |
|---|---|
| - mélange azéotrope de thioglycolate d'hydroxy-2 propyle et de thioglycolate d'hydroxy-2 méthyl-1 éthyle décrit à l'exemple 1 de FR-A-2 679 448 | 15 g |
| - monoéthanolamine qs | pH 8 |
| - eau déminéralisée qsp | 100 g |

Le mode opératoire est celui de l'exemple 1.

On obtient une mèche présentant une belle frisure.

### Exemple 7 (invention)

On utilise une composition réductrice présentant les caractéristiques suivantes :

| | |
|---|---|
| - acide mercaptopropionique | 10 g |
| - monoéthanolamine | 3 g |
| - carbonate de guanidine | 1, 5 g |
| - ammoniaque qs | pH 9 |
| - eau déminéralisée qsp | 100 g |

Le mode opératoire est celui de l'exemple 1.

On obtient finalement une mèche présentant une belle frisure.

### Exemple 8 (invention)

On utilise une composition réductrice présentant les caractéristiques suivantes :

| | |
|---|---|
| - acide thiolactique | 11 g |
| - ammoniaque | 3 g |
| - monoéthanolamine qs | pH 8 |
| - eau déminéralisée qsp | 100 g |

Le mode opératoire est celui de l'exemple 1.

On obtient finalement une mèche présentant une belle frisure.

## Revendications

1. Procédé de traitement pour la déformation permanente des matières kératiniques, caractérisé en ce qu'il comprend les étapes suivantes :
(i) on applique sur la matière kératinique à traiter une composition contenant de l'acide thioglycolique et/ou de l'acide thiolactique et/ou de l'acide mercaptopropionique et/ou un de leurs sels et/ou un de leurs esters, les moyens nécessaires à la mise sous tension mécanique de la matière kératinique étant mis en oeuvre avant, pendant ou après ladite application,
(ii) éventuellement, on soumet ensuite la matière kératinique ainsi traitée à un traitement thermique,
(iii) puis on rince la matière kératinique ainsi traitée,
(iv) on laisse ensuite reposer la matière kératinique ainsi rincée ,
(v) et enfin on sépare la matière kératinique ainsi reposée des moyens de mise sous tension utilisés à l'étape (i).

2. Procédé selon la revendication 1, caractérisé en ce que l'on utilise l'acide thioglycolique ou de l'acide thiolactique ou de l'acide mercaptopropionique ou un de leurs sels, le pH de ladite composition étant alors compris entre 6 et 11,5.

3. Procédé selon la revendication 2, caractérisé en ce que ledit pH est compris entre 7 et 10.

4. Procédé selon la revendication 1, caractérisé en ce que l'on utilise un ester de l'acide thioglycolique ou de l'acide thiolactique ou de l'acide 3-mercaptopropionique, le pH de ladite composition étant alors compris entre 5 et 11.

5. Procédé selon la revendication 4, caractérisé en ce que ledit pH est compris entre 6 et 10.

6. Procédé selon l'une quelconque des revendications précédentes, caractérisé en ce que lesdits sels sont choisis, seuls ou en mélanges, parmi les sels d'ammonium, les sels d'amines secondaire ou tertiaire, les sels alcalino-terreux.

7. Procédé selon l'une quelconque des revendications précédentes, caractérisé en ce que ledit acide thioglycolique et/ou ledit acide thiolactique et/ou ledit acide mercaptopropionique et/ou leurdits sels et/ou leurdits esters sont présents dans ladite composition dans une teneur allant de 1 à 30% en poids.

8. Procédé selon la revendication 7, caractérisé en ce que ladite teneur est comprise entre 3 et 20% en poids.

9. Procédé selon l'une quelconque des revendications précédentes, caractérisé en ce que ladite composition contient des adjuvants destinés à la rendre cosmétiquement acceptable.

10. Procédé selon la revendication 9, caractérisé en ce que lesdits adjuvants sont choisis, seuls ou en mélange, parmi des agents tensio-actifs de type non-ionique, anionique, cationique ou amphotère, des agents traitants, des ingrédients actifs, des agents antichutes, des agents anti-pelliculaires, des épaississants, des agents de suspension, des agents sequestrants, des agents opacifiants, des colorants, des filtres solaires, des parfums et des conservateurs.

11. Procédé selon l'une quelconque des revendications précédentes, caractérisé en ce que ladite composition se présente sous la forme d'une lotion, épaissie ou non, d'une crème ou d'un gel.

12. Procédé selon l'une quelconque des revendications précédentes, caractérisé en ce que, avant ladite application de ladite composition, la matière kératinique est humidifiée.

13. Procédé selon l'une quelconque des revendications précédentes, caractérisé en ce que ledit traitement thermique (étape (ii)) est conduit à une température comprise entre 30 et 60°C.

14. Procédé selon l'une quelconque des revendications précédentes, caractérisé en ce que, avant de procéder au rincage (étape (iii)), on laisse reposer (phase d'attente) la matière kératinique issue de l'étape (i), cette phase d'attente incluant l'éventuelle étape (ii).

15. Procédé selon la revendication 14, caractérisé en ce que ladite phase d'attente dure entre 2 et 30 mn, de préférence entre 5 et 20 mn.

16. Procédé selon l'une quelconque des revendications précédentes, caractérisé en ce que la phase de repos de l'étape (iv) se fait sous chauffage.

17. Procédé selon l'une quelconque des revendications précédentes, caractérisé en ce que la phase de repos de l'étape (iv) dure entre 3 et 60 mn.

18. Procédé selon la revendication 17, caractérisé en ce que ladite phase de repos de l'étape (iv) dure entre 5 et 30 mn.

19. Procédé selon l'une quelconque des revendications précédentes, caractérisé en ce que l'étape (iv) est conduite jusqu'à séchage total de la matière kératinique.

20. Procédé selon l'une quelconque des revendications 1 à 18, caractérisé en ce qu'une nouvelle opération de rinçage est conduite à l'issue de l'étape (iv) ou de l'étape (v).

21. Procédé selon la revendication 19, caractérisé en ce qu'une nouvelle opération de rinçage est conduite à l'issue de l'étape (v).

22. Procédé selon l'une quelconque des revendications précédentes, caractérisé en ce que ladite matière kératinique consiste en des cheveux.

## Claims

1. Treatment process for the permanent reshaping of keratinous matter, characterized in that it comprises the following steps:
(i) a composition containing thioglycolic acid and/or thiolactic acid and/or mercaptopropionic acid and/or one of the salts thereof and/or one of the esters thereof is applied to the keratinous matter to be treated, the means required for placing the keratinous matter under mechanical tension being employed before, during or after the said application,
(ii) if desired, the keratinous matter thus treated is subjected to a thermal treatment,
(iii) the keratinous matter thus treated is then rinsed,
(v) the keratinous matter thus rinsed is subsequently allowed to rest,
(vi) and finally the keratinous matter thus rested is separated from the means used in step (i) for placing it under tension.

2. Process according to Claim 1, characterized in that thioglycolic acid or thiolactic acid or mercaptopropionic acid or one of the salts thereof is used, the pH of the said composition then being between 6 and 11.5.

3. Process according to Claim 2, characterized in that the said pH is between 7 and 10.

4. Process according to Claim 1, characterized in that an ester of thioglycolic acid or of thiolactic acid or of 3-mercaptopropionic acid is used, the pH of the said composition then being between 5 and 11.

5. Process according to Claim 4, characterized in that the said pH is between 6 and 10.

6. Process according to any one of the preceding claims, characterized in that the said salts are chosen, individually or as a mixture, from ammonium salts, secondary or tertiary amine salts, and alkaline-earth metal salts.

7. Process according to any one of the preceding claims, characterized in that the said thioglycolic acid and/or the said thiolactic acid and/or the said mercaptopropionic acid and/or the said salts thereof and/or the said esters thereof are present in the said composition in a proportion which ranges from 1 to 30 % by weight.

8. Process according to Claim 7, characterized in that the said proportion is between 3 and 20 % by weight.

9. Process according to any one of the preceding claims, characterized in that the said composition contains adjuvants which are intended to make it cosmetically acceptable.

10. Process according to Claim 9, characterized in that the said adjuvants are chosen, individually or as a mixture, from nonionic, anionic, cationic or amphoteric surfactants, treating agents, active ingredients, agents which act against hair loss; anti-dandruff agents, thickeners, suspension agents, sequestering agents, opacifying agents, dyes, sunscreens, fragrances and preservatives.

11. Process according to any one of the preceding claims, characterized in that the said composition is in the form of a lotion, thickened or otherwise, a cream or a gel.

12. Process according to any one of the preceding claims, characterized in that the keratinous matter is moistened before the said application of the said composition.

13. Process according to any one of the preceding claims, characterized in that the said thermal treatment (step (ii)) is carried out a temperature of between 30 and 60°C.

14. Process according to any one of the preceding claims, characterized in that, before carrying out the rinsing operation (step (iii)), the keratinous matter from step (i) is left to rest (waiting phase), this waiting phase including the optional step (ii).

15. Process according to Claim 14, characterized in that the said waiting phase lasts for between 2 and 30 min, preferably between 5 and 20 min.

16. Process according to any one of the preceding claims, characterized in that the resting phase of step (iv) takes place with heating.

17. Process according to any one of the preceding claims, characterized in that the resting phase of step (iv) lasts for between 3 and 60 min.

18. Process according to Claim 17, characterized in that the said resting phase of step (iv) lasts for between 5 and 30 min.

19. Process according to any one of the preceding claims, characterized in that step (iv) is carried out until complete drying of the keratinous matter.

20. Process according to any one of Claims 1 to 18, characterized in that a further rinsing operation is carried out at the end of step (iv) or of step (v).

21. Process according to Claim 19, characterized in that a further rinsing operation is carried out at the end of step (v).

22. Process according to any one of the preceding claims, characterized in that the keratinous matter consists of hair.

## Patentansprüche

1. Behandlungsverfahren zur dauerhaften Verformung von Keratinsubstanzen, dadurch gekennzeichnet, daß es die folgenden Schritte umfaßt:
(i) Auf die zu behandelnde Keratinsubstanz wird eine Zusammensetzung aufgebracht, die Thioglykolsäure und/oder Thiomilchsäure und/oder Mercaptopropionsäure und/oder ein Salz dieser Verbindungen und/oder einen Ester dieser Verbindungen enthält, wobei die Mittel, die erforderlich sind, um die Keratinsubstanz unter Spannung zu setzen, vor, während oder nach dem Auftragen der Zusammensetzung eingesetzt werden;
(ii) die so behandelte Keratinsubstanz wird dann gegebenenfalls einer thermischen Behandlung unterzogen;
(iii) anschließend wird die so behandelte Keratinsubstanz gespült;
(iv) dann wird die so gespülte Keratinsubstanz ruhen gelassen und
(v) schließlich werden die in Schritt (i) verwendeten Mittel, mit denen die Keratinsubstanz unter Spannung gesetzt wurde, aus der so ruhen gelassenen Keratinsubstanz entfernt.

2. Verfahren nach Anspruch 1, dadurch gekennzeichnet, daß Thioglykolsäure oder Thiomilchsäure oder Mercaptopropionsäure oder ein Salz dieser Verbindungen verwendet wird, wobei der pH-Wert der Zusammensetzung im Bereich von 6 bis 11,5 liegt.

3. Verfahren nach Anspruch 2, dadurch gekennzeichnet, daß der pH-Wert im Bereich von 7 bis 10 liegt.

4. Verfahren nach Anspruch 1, dadurch gekennzeichnet, daß ein Ester von Thioglykolsäure oder Thiomilchsäure oder 3-Mercaptopropionsäure verwendet wird, wobei der pH-Wert der Zusammensetzung im Bereich von 5 bis 11 liegt.

5. Verfahren nach Anspruch 4, dadurch gekennzeichnet, daß der pH-Wert im Bereich von 6 bis 10 liegt.

6. Verfahren nach einem der vorhergehenden Ansprüche, dadurch gekennzeichnet, daß die Salze allein oder im Gemisch ausgewählt sind unter Ammoniumsalzen, Salzen von sekundären oder tertiären Aminen und Erdalkalimetallsalzen.

7. Verfahren nach einem der vorhergehenden Ansprüche, dadurch gekennzeichnet, daß die Thioglykolsäure und/oder die Thiomilchsäure und/oder die Mercaptopropionsäure und/oder die Salze dieser Verbindungen und/oder die Ester dieser Verbindungen in der Zusammensetzung in einem Mengenanteil von 1 bis 30 Gew.-% vorliegen.

8. Verfahren nach Anspruch 7, dadurch gekennzeichnet, daß dieser Mengenanteil im Bereich von 3 bis 20 Gew.-% liegt.

9. Verfahren nach einem der vorhergehenden Ansprüche, dadurch gekennzeichnet, daß die Zusammensetzung Zusatzstoffe enthält, die dazu bestimmt sind, die Zusammensetzung kosmetisch akzeptabel zu machen.

10. Verfahren nach Anspruch 9, dadurch gekennzeichnet, daß diese Zusatzstoffe allein oder im Gemisch ausgewählt sind unter grenzflächenaktiven Stoffen vom nichtionischen, anionischen, kationischen oder amphoteren Typ, Behandlungsmitteln, Wirkstoffen, Mitteln gegen Haarausfall, Mitteln gegen Schuppen, Verdickungsmitteln, Suspendiermitteln, Maskierungsmitteln, Trübungsmitteln, Färbemitteln, Sonnenschutzfiltern, Parfums und Konservierungsmitteln.

11. Verfahren nach einem der vorhergehenden Ansprüche, dadurch gekennzeichnet, daß die Zusammensetzung in Form einer gegebenenfalls verdickten Lotion, einer Creme oder eines Gels vorliegt.

12. Verfahren nach einem der vorhergehenden Ansprüche, dadurch gekennzeichnet, daß die Keratinsubstanz vor dem Auftragen der Zusammensetzung angefeuchtet wird.

13. Verfahren nach einem der vorhergehenden Ansprüche, dadurch gekennzeichnet, daß die thermische Behandlung (Schritt (ii)) bei einer Temperatur im Bereich von 30 bis 60 °C durchgeführt wird.

14. Verfahren nach einem der vorhergehenden Ansprüche, dadurch gekennzeichnet, daß die Keratinsubstanz nach dem Schritt (i) ruhen gelassen wird (Wartephase), bevor gespült wird (Schritt (iii)), wobei diese Wartephase Bestandteil des gegebenenfalls durchgeführten Schritts (ii) ist.

15. Verfahren nach Anspruch 14, dadurch gekennzeichnet, daß die Wartephase 2 bis 30 Minuten und vorzugsweise 5 bis 20 Minuten dauert.

16. Verfahren nach einem der vorhergehenden Ansprüche, dadurch gekennzeichnet, daß während der Ruhephase in Schritt (iv) erwärmt wird.

17. Verfahren nach einem der vorhergehenden Ansprüche, dadurch gekennzeichnet, daß die Ruhephase in Schritt (iv) 3 bis 60 Minuten dauert.

18. Verfahren nach Anspruch 17, dadurch gekennzeichnet, daß die Ruhephase in Schritt (iv) 5 bis 30 Minuten dauert.

19. Verfahren nach einem der vorhergehenden Ansprüche, dadurch gekennzeichnet, daß in Schritt (iv) ruhen gelassen wird, bis die Keratinsubstanz völlig trocken ist.

20. Verfahren nach einem der Ansprüche 1 bis 18, dadurch gekennzeichnet, daß nach Schritt (iv) oder Schritt (v) ein erneuter Spülvorgang durchgeführt wird.

21. Verfahren nach Anspruch 19, dadurch gekennzeichnet, daß nach Schritt (v) ein erneuter Spülvorgang durchgeführt wird.

22. Verfahren nach einem der vorhergehenden Ansprüche, dadurch gekennzeichnet, daß es sich bei der Keratinsubstanz um Haare handelt.
